# EUROPÄISCHE PATENTSCHRIFT

(11) **EP 0 386 076 B1**
(45) Veröffentlichungstag und Bekanntmachung des Hinweises auf die Patenterteilung: **03.02.1993**
(21) Anmeldenummer: 88909758.0
(22) Anmeldetag: 04.11.1988
(51) Int. Cl.: C07K 7/10, C07K 13/00

(54) **PHOSPHORYLIERTE DERIVATE VON CARDIODILATIN/ANF-PEPTIDEN**
PHOSPHORYLED DERIVATIVES OF CARDIODILATINE/ANF PEPTIDES
DERIVES PHOSPHORYLES DE PEPTIDES DE FNA/CARDIODILATINE

(30) Priorität: 07.11.1987 DE 3737917
(43) Veröffentlichungstag der Anmeldung: 12.09.1990
(73) Patentinhaber: FORSSMANN, Wolf-Georg, Prof. Dr. med., D-69121 Heidelberg (DE); PHARMA BISSENDORF PEPTIDE GMBH, 30625 Hannover (DE)
(72) Erfinder: FORSSMANN, Wolf-Georg, D-6900 Heidelberg 1 (DE); GAGELMANN, Michael, D-6905 Schriesheim (DE); HOCK, Dieter, D-6924 Neckarbischofsheim (DE)
(74) Vertreter: Werner, Hans-Karsten, Dr.Dipl.-Chem.
(86) Internationale Anmeldenummer: EP8801000
(87) Internationale Veröffentlichungsnummer: WO8904324

(56) Entgegenhaltungen:
- EP-A- 0 182 984
- DE-A- 3 443 257
- US-A- 4 508 712
- Chemical Abstracts, vol. 105, Nr. 10, 10 March 1986 (Columbus, Ohio, US), Rittenhouse, Judith et al.: Phosphorylation of atrial natriuretic peptides by cyclic AMP-dependent protein Kinase", see page 138, abstract 73295w, & J. Biol. Chem. 1986, 261(17), 7607-1
- Chemcial Abstracts, vol. 108, Nr. 7, 155 February 1988, (Columbus, Ohio, US), Olins Gillian M et al.: "Phosphorylation of high- and low-molecular-mass atrial natriuretic peptide analogs by cyclic AMP-dependent protien Kinase.", see page 148, abstract 49972f, & FEBS Lett. 1987, 224(2), 323-3
- Chemical Abstracts, vol. 108, Nr. 17, 25 April 1988, (Columbus, Ohio, US), Rittenhouse, Judith et al.: Phosphorylation in situ of atrial natriuretic peptide prohormone at the cyclic AMP-dependent site.", see page 156, abstract 144184j, & J. Biol. Chem. 1988, 263(8), 3778-8
- Chemical Abstracts, vol. 107, Nr. 15, 12 October 1987, (Columbus, Ohio, US), Bloch, Kenneth D. et al.: Proatrial natriuretic factor is phosphorylated by rat cardiocytes in culture.", see page 151, abstract 127948y, & J. Biol. Chem. 1987, 262(21), 9956-6

## Beschreibung

Die Erfindung betrifft Derivate des Precursor-Peptides des Cardiodilatin/atrialen-natriuretischen Faktors pre-pro-Gamma-hANaP (CDD-ANF) oder Fragmente desselben, die zumindest die Aminosäuresequenz des Alpha-hANaP aufweisen. Des weiteren betrifft die Erfindung ein Verfahren zur Herstellung der genannten Derivate sowie ihre Verwendung.

Cardiodilatin, Alpha-hANaP und weitere natriuretisch/diuretisch wirksame Peptide stammen aus einem gemeinsamen Precursor-Peptid, dem pre-pro-Gamma-hANaP ab, welches 151 Aminosäuren umfaßt.

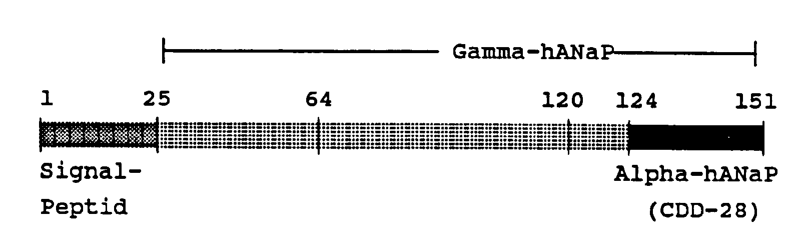

Die Positionen 1-25 der Aminosäuresequenz werden als Signal-Peptid angesehen, während die Positionen 25-151 das Gamma-hANaP darstellen, welches unter anderem im Bereich des C-terminalen Endes (Positionen 124-151) mit dem Alpha-hANaP identisch ist (Oikawa, S. et al., "Nature" 309 (1984), 724-726; Nakayama, K. et al., "Nature" 310 (1984), 699-701). Die Numerierung der Aminosäurepositionen, die im folgenden verwendet wird, berücksichtigt das Signal-Peptid jedoch nicht. Somit gilt für Gamma-hANaP eine Aminosäuresequenz von Position 1-126 und für Alpha-hANaP von 99-126 (CDD-28).

Es sind weitere Peptidfragmente des Gamma-hANaP beschrieben, nämlich Fragmente mit den Aminosäuren 39-126 (CDD-88) und Aminosäuren 95-126 (CDD-32). Letzteres besitzt an N-Terminus 4 Aminosäuren mehr als CDD-28 und wird auch als Urodilatin bezeichnet. Den atrialen Peptiden werden blutdruckregulierende Wirkungen durch Stimulation von Natriurese, Diurese und Relaxation der glatten Muskulatur zugeschrieben. Die DE-OS 34 43 257 beschreibt Alpha-hANaP als Mittel zur Behandlung verschiedener Krankheiten wie Herzinsuffizienz, oligurischem Nierenversagen, Blutdruckdisregulation und Aszites.

Peptide, insbesondere körpereigene, werden in vivo durch enzymatische Reaktionen abgebaut. Es ist deshalb nur schwer möglich, über einen langen Zeitraum die entsprechende therapeutisch wirksame Dosierung zu verabreichen, insbesondere, weil sich eine den Abbau des Peptids kompensierende, höhere Dosierung wegen der hohen physiologischen Wirksamkeit der körpereigenen Botenstoffe in den meisten Fällen verbietet. Wünschenswert ist also die Bereitstellung einer Form des biologisch aktiven Peptides, in welcher es möglichst geringe biologische Aktivität aufweist, aber durch körpereigene, unbedenkliche Modifizierungsreaktionen in die physiologisch aktive Form überführt wird.

Wegen der Bedeutung des Cardiodilatins bzw. atrialennatriuretischen Faktors ist es wichtig, ein Verfahren zur sicheren analytischen Erfassung der aktuellen Konzentration der entsprechenden physiologisch wirksamen Verbindung zur Verfügung zu haben, um die wirksamen Peptide und/oder Peptidfragmente qualitativ und quantitativ zu erfassen.

Ein bekanntes biochemisches Prinzip der Modulation der Wirkung von Biomolekülen, wie Enzymen und Rezeptoren, beruht auf Phosphorylierungs- und Dephosphorylierungsreaktionen.

Rittenhouse, J. et al., J. Biol. Chem. 261 (1986), 7607-7610, beschreibt die Phosphorylierung von synthetischen Peptiden mit der Aminosäuresequenz Arg¹⁰¹-Tyr¹²⁶ des Alpha-hANaP mittels c-AMP-abhängiger Proteinkinase, die als Erkennungssequenz Arg-Arg-X-Ser erfordert. Es wird dort ebenfalls beschrieben, daß das synthetische Peptid eine Michaeliskonstante von Kₘ = 0.5 µM aufweist. Das an Ser¹⁰⁴ in vitro phosphorylierte synthetische Peptid besitzt eine physiologische Aktivität, die sich in der Stimulierung des Na/K/Cl-Cotransportes in kultivierten vasculären Zellen der glatten Muskulatur äußert. Bloch, K.D. et al., J. Biol. Chem. 262 (1987), 9956-9961, beschreibt hingegen Versuche, wobei, in vivo in Primärkulturen von Cardiozyten neugeborener Ratten, ein Serinrest in einem durch Trypsinabbau erhaltenen Fragment (Positionen 26-67 des Gamma-hANaP) des pro-ANF (Gamma-hANaP) phosphoryliert wird. Diese Befunde sprechen also dafür, daß Gamma-hANaP nicht in dem Bereich phosphoryliert wird, welcher als Alpha-hANaP abgespalten wird. Damit kommt den synthetischen phosphorylierten Peptiden Arg¹⁰¹-Tyr¹²⁶ bzw. Gly⁹⁶-Tyr¹²⁶ (Rittenhouse et al.) keine natürliche physiologische Bedeutung zu.

Die der Erfindung zugrunde liegende Aufgabe besteht darin, chemische Verbindungen bereitzustellen, die eine einfache qualitative und quantitative Bestimmung von Peptiden, die die Sequenz des wirksamen Prinzips des Gamma-hANaP, nämlich das Alpha-hANaP (Positionen 98-126) enthalten, erlauben. Des weiteren liegt der Erfindung die Aufgabe zugrunde, chemische Verbindungen bereitzustellen, aus denen durch die körpereigene Modifikation ein Wirkstoff entsteht, wobei die Stammverbindung selbst keine oder nur geringere physiologische Aktivität aufweist. Des weiteren besteht eine Aufgabe der Erfindung darin, ein Verfahren bereitzustellen, mit dessen Hilfe die Anwesenheit von natriuretischen-atrialen Peptiden qualitativ und quantitativ erfaßt werden kann.

Die der Erfindung zugrunde liegenden Aufgaben werden gelöst durch eine Verbindung mit der Formel I 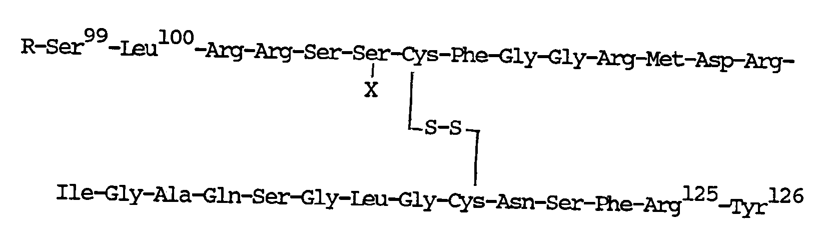
wobei X eine Phosphatgruppe oder eine Thiophosphatgruppe und R entweder ein -H Rest oder ein Peptidfragment aus der Aminosäuresequenz des Gamma-hANaP ist.

Die erfindungsgemäße Verbindung der Formel I kann in besonders eleganter Weise durch enzymatische Phosphorylierung der entsprechenden Ausgangsverbindung der Formel II 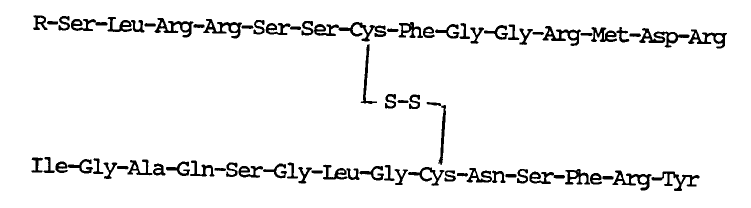
mit den entsprechenden Nucleotiden erreicht werden. Gemäß einer bevorzugten Ausführungsform wird als Enzym c-AMP-abhängige Proteinkinase oder deren katalytische Untereinheit und als Nucleotid Adenosin-5′-triphosphat (ATP) bzw. Adenosin-5′-(Gamma-thio)triphosphat mit der Verbindung der Formel II in an sich bekannter Weise umgesetzt. Es können auch radioaktiv markierte Nucleotide eingesetzt werden. Dabei werden üblicherweise ³²P oder, im Falle der Verwendung von (Gamma-thio)-Nucleotiden, ³⁵S aufweisende Nucleotide eingesetzt. So erhält man die entsprechenden radioaktiv markierten Peptide, die mindestens die Aminosäuresequenz des Alpha-hANaP (CDD-28) enthalten.

Es ist natürlich auch möglich, mittels der dem Fachmann bekannten Merrifield-Synthese durch Verwendung entsprechender phosphorylierter Bausteine, zum Beispiel von phosphoryliertem Serin, sei es in radioaktiv markierter oder nicht markierter Form, die erfindungsgemäßen Verbindungen herzustellen.

Gemäß einer bevorzugten Ausführungsform umfaßt das Peptidfragment R die Aminosäuren 1-98 (Gamma-hANaP), 39-98 (CDD-88) und 95-98 (CDD-32), auch als Urodilatin bezeichnet.

Ein analytisches Verfahren zur qualitativen und/oder quantitativen Bestimmung natriuretischer Peptide, die von pre-pro-Gamma-hANaP abgeleitet sind, ist mit den entsprechenden radioaktiv markierten phosphorylierten Peptiden möglich. Gemäß der Erfindung werden solche Peptide direkt erfaßt, die mindestens die Alpha-hANaP (CDD-28) -Sequenz besitzen. Die analytische Erfassung der physiologisch wirksamen Peptide des pre-pro-Gamma-hANaP ist von diagnostischem Interesse, um aufgrund von Durchschnittswerten abweichender Konzentrationen in Körperflüssigkeiten, zum Beispiel im Blutserum und Urin, bestimmte Krankheitsbilder zu differenzieren. Zu diesem Zweck kann zum Beispiel ein in der Körperflüssigkeit vorhandenes Peptid der Formel II umgesetzt werden mit c-AMP-abhängiger Proteinkinase oder deren katalytischer Untereinheit in Gegenwart von radioaktivem Nucleotid, wie beispielsweise (Gamma-³²P]ATP oder ³⁵S-bzw. ³²P-markiertem Adenosin-5′-(Gamma-thio)triphosphat. In dieser Ausführungsform werden alle physiologisch wirksamen Fragmente des pre-pro-Gamma-hANaP markiert, die zumindest die CDD-28-Sequenz aufweisen. Zur Durchführung des Analyseverfahrens wird vorzugsweise die Probe bestehend aus Körperflüssigkeiten oder Gewebehomogenaten in geeigneter Weise vorbereitet, indem sie beispielsweise im ersteren Fall durch eine unpolare Matrix gedrückt werden.

Danach werden die Proben mit dem entsprechenden Nucleotid in Gegenwart eines Enzyms, wie c-AMP-abhängiger Proteinkinase, phosphoryliert. Das markierte Fragment oder pre-pro-Gamma-hANaP selbst wird mittels bekannter Methoden wie PAGE-Elektrophorese, Harnstoffgel-Elektrophorese mit anschließender Autoradiographie bzw. Szintillationsmessung, Dünnschichtchromatographie, Hochspannungs-Elektrophorese, isoelektrische Fokussierung oder auch HPLC (High Performance Liquid Chromatography) direkt nachgewiesen. Grundsätzlich ist es jedoch auch möglich, die mit Hilfe der markierten phosphorylierten Peptide isolierten Fraktionen durch andere biochemische oder immunologische Methoden (monoklonale Antikörper) näher zu charakterisieren.

Die Nachweisgrenze läßt sich entweder durch Zusatz größerer Mengen des Enzyms, wie c-AMP-abhängiger Proteinkinase oder deren katalytischer Untereinheit, bzw. durch Verlängerung der Inkubationsdauer sowie durch Verwendung von Nucleotiden mit höherer spezifischer Radioaktivität erhöhen. Es besteht ein erheblicher zeitabhängiger Verlust von Peptidmaterial wie zum Beispiel Alpha-hANaP/CDD-28 durch Adsorption des Materials an Glasoberflächen, der durch Verwendung von Polyethylenreaktionsgefäßen stark vermindert wird. Die von c-AMP abhängige Phosphorylierung kann die biologische Aktivität von Peptidmaterial verändern. Bei zusätzlichem Nachweis von Substanzen durch einen Bioassay empfiehlt sich daher unter Umständen die alleinige Verwendung von [Gamma-³²P]ATP als Kosubstrat ohne entsprechendes nicht markiertes ATP.

Die Verwendung der Verbindung der Formel I führt in einer bevorzugten Ausführungsform der Erfindung zur qualitativen und/oder quantitativen Erfassung von Alpha -hANaP (CDD-28), CDD-32, CDD-88 und Gamma-hANaP sowie auch pre-pro-Gamma-hANaP.

Derivate der erfindungsgemäßen Verbindung der Formel I können wegen ihrer physiologischen Wirkung als Arzneimittel eingesetzt werden. In den Fällen, in denen die Verbindungen gemäß Formel I einen geringeren physiologischen Effekt aufweisen als die korrespondierenden eigentlichen Wirkstoffe, können die erfindungsgemäßen Verbindungen dazu benutzt werden, den eigentlichen Wirkstoff nach Formel II als eine Art Depotform zu speichern. In vivo werden sukzessive die Verbindungen der Formel I infolge der Einwirkung von Hydrolasen, inbesondere dephosphorylierender Enzyme (Phosphatasen), in die aktive Form überführt.

Diese biologischen Wirkungen zeigen, daß das phosphorylierte Peptid gemäß Formel I eine große klinische, diagnostische und therapeutische Bedeutung besitzt. Insbesondere eignet sich die Verbindung gemäß Formel I für die folgenden Anwendungsbereiche: differenzierte Vasodilation bestimmter Gefäßbetten, Diagnostik und Therapie der Hypertonie, Anwendung als Substitution bei Patienten, denen künstliche Herzen implantiert wurden, synchrone Regulation des Blutvolumens und der Blutelektrolyte, Hauterkrankungen, insbesondere mit Störungen der Schweißsekretion, cardiovaskulärer Schock, Erkrankungen der Nieren und Nebennierenrinde, Erkrankungen des Gastrointestinaltraktes, insbesondere bei Motilitätsstörungen bzw. Obstipation, Therapie von Hirnödemen und des Glaukoms, Therapie spastischer Coronaropathien wie Angina pektoris, zur Behandlung von akuter Niereninsuffizienz, nephrotischem und nephritischem Syndrom, terminaler Niereninsuffizienz, akuter Herzinsuffizienz, Ascites, generalisierten Ödemen, Lungenödem, Hirnödem, primären und sekundären Lymphödemen, Hydrothorax, Glaukom, Vena-cava-Stenose, Hypoproteinämie, Hörsturz, essentieller und renaler Hypertonie, maligner Hypertonie, EPH-Gestose wie Schwangerschaftshypertonie, Schwangerschafts-Nephrose, zerebralem Natriumspeichersyndrom, Gefäßspasmen wie Morbus Raynaud, Angina abdominalis, Koronarspasmen, koronarer Herzkrankheit, vasomotorischen Kopfschmerzen, Kopfschmerzen bei Hypertonie, Bing-Horton-Syndrom Migräne, Durchblutungsstörungen im Vertebralis-Basilaris-Strombahngebiet, Störungen des Renin-Angiotensin-Systems, primärem und sekundärem Hyperaldosteronismus, reninsezernierenden Tumoren, Phäochromozytom, Bartters Syndrom, Schwartz-Bartter-Syndrom, Pankreasinsuffizienz, primärer Schweißdrüseninsuffizienz wie Anhidrosis, Miktions- und Defäkationsstörungen, Hypophysenvorderlappendysregulation, cardio-vaskulären Nebenwirkungen bei der Therapie psychiatrischer Erkrankungen sowie Agitation mit Catecholaminerhöhung, zur unterstützenden Behandlung bei Herztransplantationen, "PEEP-Beatmung" (einer mechanischen Beatmung mit positivem endexpiratorischem Druck) sowie Bypass-Operationen und zur Behandlung von Komplikationen nach Einpflanzung eines künstlichen Herzens.

Die Verbindung gemäß Formel I kann ebenfalls eingesetzt werden als Diuretikum zur Behandlung von multiplen Erkrankungen wie Gicht, kombiniert mit Hypertonus/Herzinsuffizienz sowie Diabetes mit Hypertonus/Herzinsuffizienz oder als Diuretikum in Kombination mit Cephalosporinen, Aminoglykosiden oder Antikoagulantien. Des weiteren eignet sich diese Verbindung zur Prophylaxe von akutem Nierenversagen nach Nierentransplantationen und Behandlung mit nephrotoxischen Substanzen wie Cyclosporin, Cisplatin und Ifosfamid und zur Prophylaxe des akuten Nierenversagens, postoperativ oder nach hypertonen Krisen und nach Gabe von Kontrastmitteln bei Patienten mit eingeschränkter Nierenfunktion. Auch als Diagnostikum zur Differentialdiagnose von Endothel-Veränderungen ist die erfindungsgemäße Verbindung einsetzbar.

Gegenstand der Erfindung sind deshalb auch Arzneimittel zur Verwendung bei den oben angegebenen Diagnose- und Therapieverfahren, welche die phosphorylierte Verbindung gemäß Formel I enthalten. Die Arzneimittel können in den üblichen Anwendungsformen zur intravenösen, oralen oder parenteralen Applikation vorliegen, wie z.B. als Tabletten, Suppositorien, Dragées, Lösungen, Sprays oder Zubereitungen zur Inhalation usw., sowie Mikroverkapselung, gegebenenfalls zusammen mit üblichen, pharmakologisch verträglichen Träger- und/oder Verdünnungsmitteln. Das erfindungsgemäße Medikament kann auch so formuliert werden, daß der Wirkstoff langsam freigesetzt wird. Vorzugsweise beträgt die Menge des Wirkstoffes 50 ng bis 50 µg pro Dosiseinheit.

Weiterhin wird erfindungsgemäßen ein Verfahren zur spezifischen Bestimmung der Verbindung gemäß Formel I zur Verfügung gestellt, bei dem man nach dem Prinzip des Immunoassays arbeitet und einen Antikörper, der gegen diese Verbindung gerichtet ist, verwendet. Es wurde festgestellt, daß durch Verwendung von Antikörpern gegen die Verbindung gemäß Formel I auch Cardiodilatin in Körperflüssigkeiten sehr spezifisch und sehr genau nachgewiesen werden kann. Bevorzugt kann dieses Verfahren zur spezifischen Bestimmung von Cardiodilatin verwendet werden zur Diagnose von neurologischen Erkrankungen, indem im Liquor cerebrospinalis Cardiodilatin oder dessen Fragmente spezifisch nachgewiesen werden.

Die Verwendung des erfindungsgemäßen Arzneimittels, welches als wirksame Substanz die Verbindung der Formel I enthält, ist aufgrund seines günstigen Wirkungsspektrums und seiner geringen Toxizität zur Behandlung von Erkrankungen angezeigt, die mit einer Störung des Elektrolyt- und Wasserhaushaltes einhergehen, wie zum Beispiel präterminale und terminale Niereninsuffizienz, Störungen des Renin-Angiotensin-Aldosteron-Systems, Störungen der Vasopressin-Sekretion, bei Flüssigkeitssequestration ("Third Space"-Probleme wie Ascites, Glaukom, Hirnödem, Hydrothorax) sowie anderen Erkrankungen mit erhöhter extravasaler Volumenansammlung wie Lymphödem, EPH-Gestose, Vena-cava-Stenose, Erkrankungen mit Hypoproteinämie wie Nierenerkrankungen, Enteropathien und Lebererkrankugen.

Das erfindungsgemäße Arzneimittel kann aufgrund seiner vasodilatatorischen Eigenschaften zur Behandlung aller Erkrankungen verwendet werden, die mit einer erhöhten Vasokonstriktion einhergehen. So kann das die erfindungsgemäße Verbindung enthaltende Arzneimittel eingesetzt werden zur Behandlung von Gefäßspasmen wie zum Beispiel Morbus Raynaud, Spasmen der muskulären Verteilerarterien und Hörsturz. Auch für weitere Gefäßerkrankungen kann die Verbindung der Formel I eingesetzt werden, wie zum Beispiel für die Behandlung von koronarer Herzkrankheit, Angina abdominalis, vasomotorischen Kopfschmerzen, Arteria basilaris Migräne mit Durchblutungsstörungen im Vertebralis-Basilaris-Strombahngebiet, Bing-Horton-Syndrom.

Aufgrund seiner blutdrucksenkenden Eigenschaften, die vor allem bei primär erhöhtem Blutdruck wirksam werden, ist das die Verbindung der Formel I enthaltende erfindungsgemäße Arzneimittel indiziert bei maligner Hypertonie, Schwangerschaftshypertonie, Kopfschmerz bei Hypertonie, Hypertonie in Kombination mit anderen Erkrankungen, zum Beispiel Gicht und Diabetes.

Das erfindungsgemäße Arzneimittel zeigt antagonistische Eigenschaften gegen natriumretinierende, wasserretinierende und vasokonstriktorische Hormone. Dies ermöglicht die Verwendung des erfindungsgemäßen Arzneimittels zur Behandlung von primärem und sekundärem Hyperaldosteronismus, renovaskulärer Hypertonie, reninsezernierenden Tumoren, Phäochromozytom, Bartter-Syndrom und Schwartz-Bartter-Syndrom.

Das die Verbindung der Formel I enthaltende erfindungsgemäße Arzneimittel kann weiterhin verwendet werden zur Behandlung der Herzinsuffizienz, bei Herztransplantationen und künstlichem Herzen, zur Unterstützung bei "PEEP-Beatmung (positive end expiratory pressure), während Bypass-Operationen und Operationen am offenen Herzen. Bei der Beatmung mit positiv-endexpiratorischem Druck erfolgt die Expiration gegen einen Überdruck.

Das erfindungsgemäße Arzneimittel kann zur Prophylaxe von akutem Nierenversagen, zum Beispiel nach Nierentransplantationen, postoperativ und zur Funktionsverbesserung bei Transplantatnieren durch vorherige Behandlung mit dem erfindungsgemäßen Arzneimittel verwendet werden.

Das die Verbindung der Formel I enthaltende erfindungsgemäße Arzneimittel kann ebenfalls zu diagnostischen Zwecken verwendet werden. Da die Vasodilatation endothel-unabhängig ist, läßt ein Vergleich mit der Vasodilatation durch einen endothel-abhängigen Vasodilatator eine Aussage über den Zustand des Endothels zu.

Die Verwendung des erfindungsgemäßen Arzneimittels ist auch bei gastrointestinalen Indikationen wie Veränderung der Pankreassekretion und damit verbundenen Verwertungsstörungen, Mangelernährungen, exokriner Pankreasinsuffizienz, Regulation der Motilität des Darmes und der Blase (Miktions- und Defäkationsstörungen) angezeigt.

In der Dermatologie kann das erfindungsgemäße Arzneimittel zur Behandlung von Anhidrosis verwendet werden. Auch in der Psychiatrie kann das erfindungsgemäße Mittel zur Behandlung von cardiovaskulären Nebenwirkungen bei der Therapie psychiatrischer Erkrankungen sowie Agitation mit Catecholaminausschüttung Verwendung finden.

Die Effekte werden bereits bei Konzentrationen im nanomolaren Bereich beobachtet. Aufgrund der geringen Toxizität und guten Verträglichkeit können aber auch höhere Konzentrationen injiziert oder infundiert werden.

Die Verbindung der Formel I kann zur Herstellung von Arzneimitteln wie Injektions- oder Infusionslösungen, als Nasenspray, Augentropfen oder als Slow-Release-Form bei den oben genannten Indikationen verwendet werden.

Die Prüfung der akuten Toxikologie am Tier hat gezeigt, daß in einem Dosisbereich bis zu 400 µg/kg Körpergewicht keine toxischen Reaktionen erfolgen, die mit üblichen Kontrollparametern (Biochemie) erfaßbar sind. Ein LD₅₀-Wert ist aufgrund der geringen Toxizität der Substanz nicht bestimmbar. Es wurden keine ernsten Nebenwirkungen am Herzkreislaufsystem, im Bronchialsystem, der Leber- und Nierenfunktion, an den Keimdrüsen und dem Zentralen Nervensystem gefunden.

Die pharmakologische Wirksamkeit bei der Behandlung der oben genannten Erkrankungen ergibt sich aus der beobachteten natriuretischen Wirkung, die bei intravenöser Applikation zu einem Anstieg der Diurese und Natriumexkretion führt. Weiterhin wurde beobachtet, daß die Verbindung der Formel I in der Lage ist, den vasokonstriktorischen Effekt von Noradrenalin aufzuheben.

Weiterhin wurde beobachtet, daß die Substanz die gefäßkontrahierende Wirkung von Angiotensin hemmt. Von besonderem Interesse sind Fälle von schwerer Herzinsuffizienz mit generalisierter Oedematose, die auf konventionelle Therapie nicht ansprechen. Von erheblicher Bedeutung ist auch die Einwirkung der Substanz auf die Blutdruckregelsysteme und die Vasopressinregelsysteme, so daß auch Funktionsstörungen des Hypophysenhinterlappens und hierdurch bedingte psychotrope Effekte indiziert sind. Es ist nicht auszuschließen, daß die intensivere klinische Untersuchung dieser Substanz weitere interessante Indikationen ergibt.

In einer bevorzugten Ausgestaltung der Erfindung können Thiophosphatgruppen aufweisende Peptide in besonders vorteilhafter Weise als Depotform eingesetzt werden, da diese Derivate nur äußerst langsam in die entsprechenden physiologisch wirksamen Äquivalente transformiert werden.

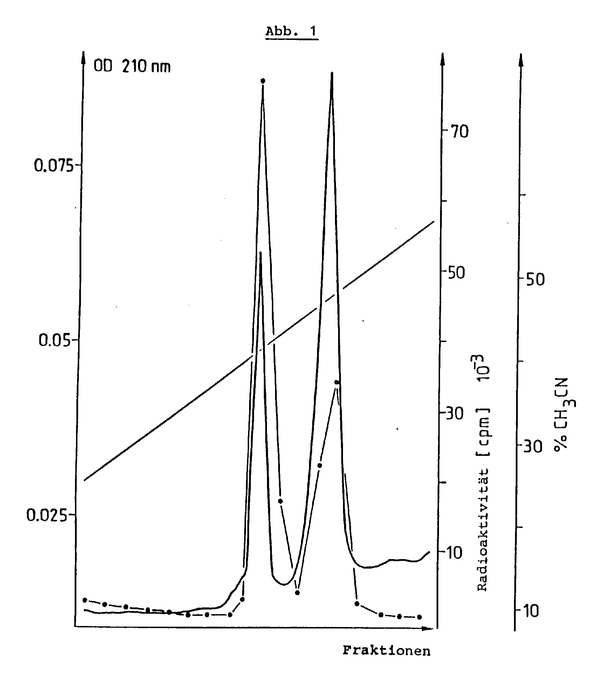

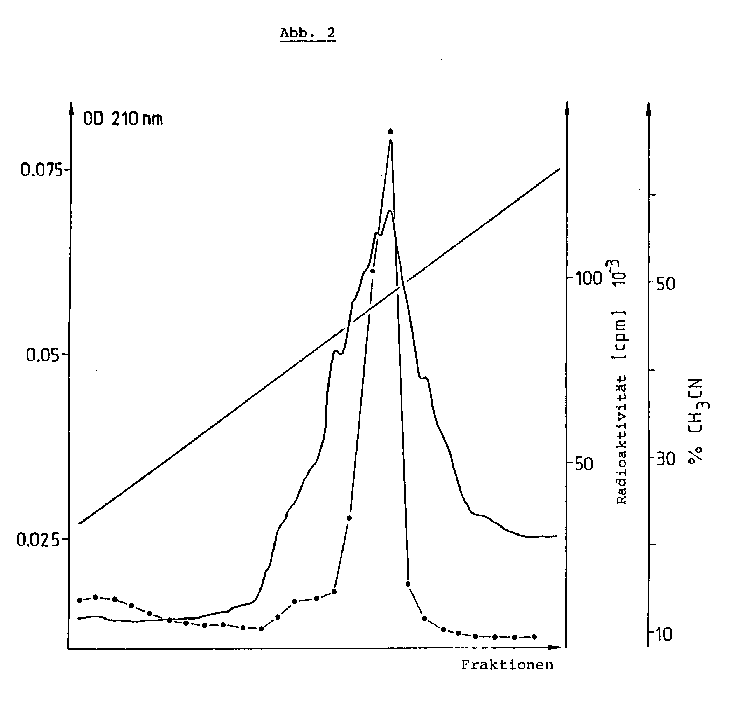

Die Abbildung 1 zeigt ein Elutionsmuster von phosphoryliertem Alpha-hANaP (CDD-28) und CDD-88. Die Abbildung 2 zeigt ein Elutionsmuster von phosphoryliertem atrialen Rinderrohextrakt. Dabei wurden Aliquots von CDD-28 und CDD-88, die mit der katalytischen Untereinheit der c-AMP-abhängigen Proteinkinase phosphoryliert worden waren, mittels Umkehrphasen-HPLC (reversed phase HPLC) an einer TSK-ODS-120T-Säule (300 x 7,8 mm ID) getrennt. Als Elutionsmittel dienten a) 0,01 M Salzsäure, b) 0,01 M Salzsäure in 80%-igem wäßrigen Acetonitril. Die Fließgeschwindigkeit betrug 1,5 ml/min. Der Lösungsmittelgradient wird durch die schräg verlaufende, durchgezogene Linie bezeichnet. Die optische Dichte (――) wurde bei 210 nm gemessen (Absorptionsbereich 0,1). Für die Messung der Radioaktivität (·――·) wurden 0,5 ml von jeder der gesammelten Fraktionen entnommen und gezählt.

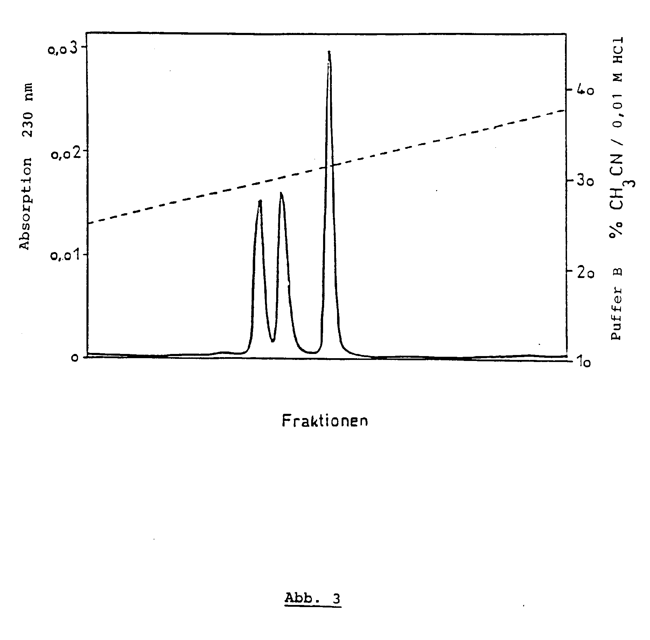

Die Abbildung 3 zeigt die Trennung von jeweils 2 µg unphosphoryliertem Alpha-hANaP (CDD-28), CDD-32 (Urodilatin) sowie phosphoryliertem Alpha-hANaP. Die Proben wurden gemischt und mittels RP-HPLC (Umkehrphasen-HPLC) getrennt. Das Elutionsmittel bestand aus einem linearen Gradienten aus Acetonitril/0,01% HCl als Puffer B und 0,01% HCl als Puffer A. Die Anfangskonzentration an Puffer B betrug 20%; die Säule wurde unter kontinuierlicher Zugabe von Puffer B (0,5%/min) mit einer Fließgeschwindigkeit von 0,7 ml/min entwickelt. Als Signal wurde die Absorption bei 230 nm gemessen. Die unter diesen Bedingungen durchgeführte Trennung führt zunächst zur Elution von nicht phosphoryliertem Alpha-hANaP, gefolgt von phosphoryliertem Alpha-hANaP und schließlich zur Elution von CDD-32 (Urodilatin).

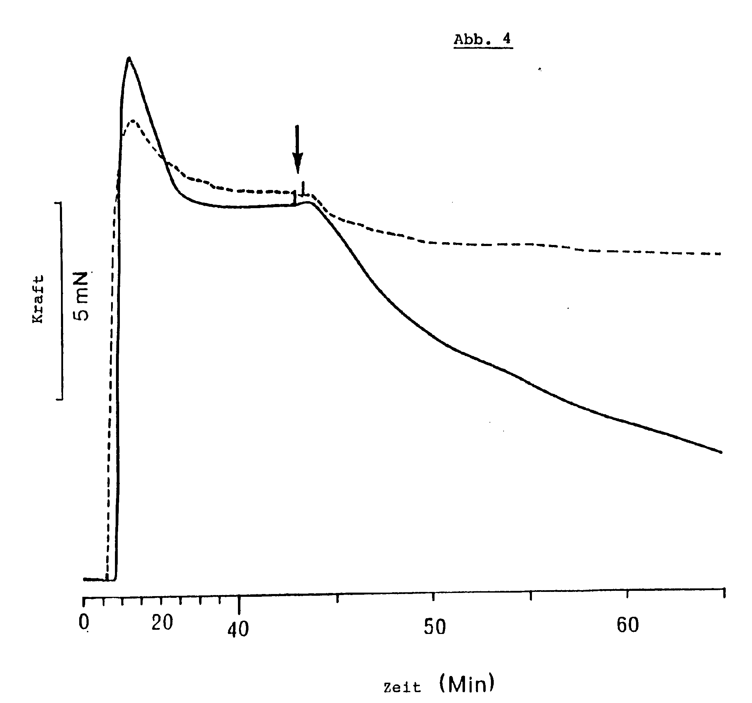

Die Abbildung 4 zeigt die Wirkung von c-AMP-abhängig phosphoryliertem (---) und nicht phosphoryliertem (――) CDD-28 auf die glatte Gefäßmuskulatur. Muskelstreifen von Kaninchenaorta wurden zunächst mit einer Lösung von Norepinephrin (10⁻⁷ M) kontrahiert. Nachdem sich konstante Bedingungen eingestellt hatten, wurden die Peptide hinzugefügt (Position des Pfeils). Die Endkonzentration belief sich auf 0,3 bis 0,6 x 10⁻⁹ M. Die durchgezogene Linie zeigt das typische Relaxationsverhalten mit nicht phosphoryliertem CDD-28. Die gestrichelte Linie dagegen demonstriert das Verhalten nach Applikation von phosphoryliertem CDD-28. Unter diesen Bedingungen ist nur eine geringfügige Relaxation nachweisbar.

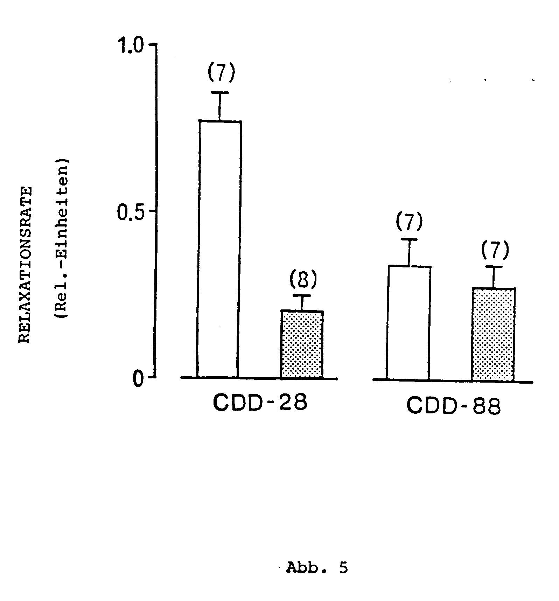

Die Abbildung 5 ist die Zusammenfassung einer Anzahl von Experimenten mit phosphoryliertem CDD-28 und CDD-88, sowie unphosphoryliertem CDD-28 und CDD-88. In beiden Fällen betrug die Konzentration 0,3 bis 0,6 x 10⁻⁹ M. In der Darstellung ist die relative Rate der Erschlaffung sowie der Standardfehler für nicht phosphorylierte (offene Rechtecke) sowie phosphorylierte (getönte Rechtecke) CDD-28- bzw. CDD-88-Peptide gezeigt (die Zahlen über den Rechtecken entsprechen der Anzahl der durchgeführten Versuche).

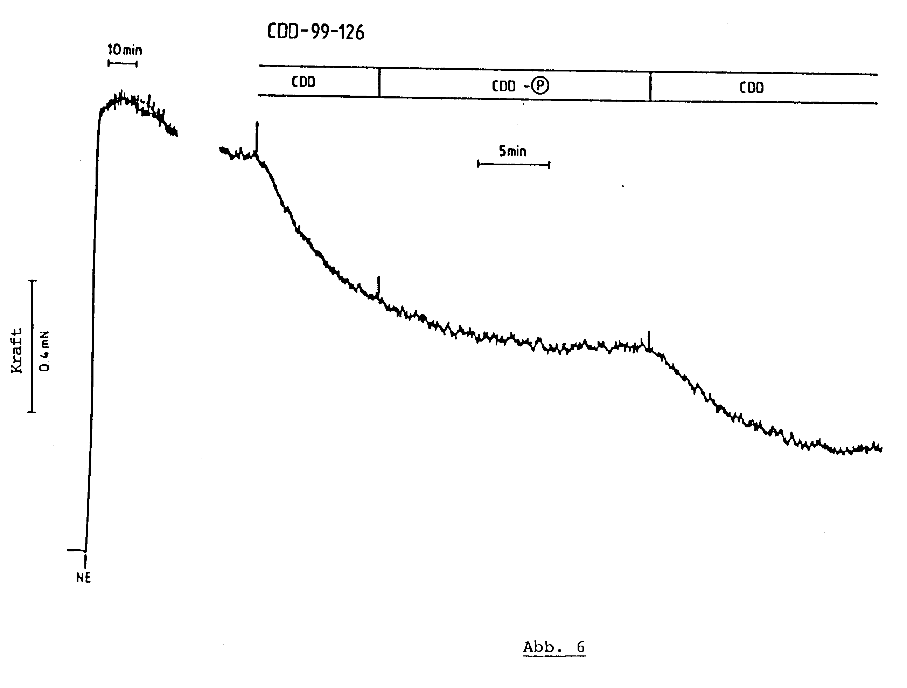

Die Abbildung 6 zeigt die Reaktion von Kaninchenaorta-Muskelstreifen auf die schrittweise Zugabe von nicht phosphoryliertem, phosphoryliertem und danach wieder nicht phosphoryliertem Alpha-hANaP (CDD-28). Die Verbindungen wurden jeweils so zugegeben, daß sie im Untersuchungsbad in einer Endkonzentration von 0,3 x 10⁻⁹ M vorlagen. Vorkontrahierte Aorta-Muskelstreifen relaxieren bei Zugabe von nicht phosphoryliertem Alpha-hANaP zu einem gewissen Grad. Die Zugabe von phosphoryliertem Alpha-hANaP (CDD ∼ Ⓟ) zeigt jedoch so gut wie keine Wirkung. Erst nach Zugabe von weiterem nicht phosphoryliertem Alpha-hANaP schreitet die Relaxation des Muskels weiter fort.

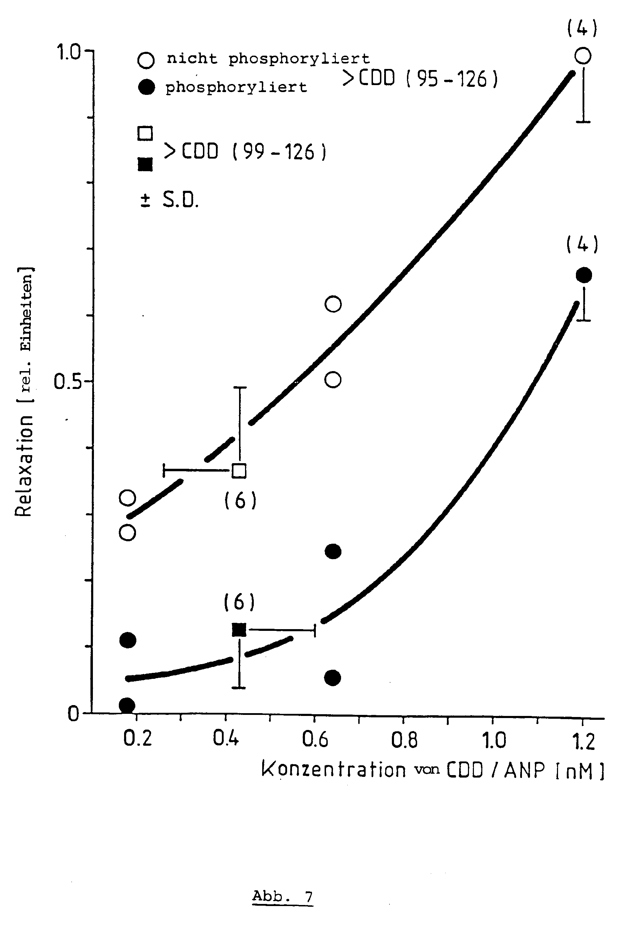

Die Abbildung 7 zeigt das relative Relaxationsvermögen von phosphoryliertem und nicht phosphoryliertem CDD-32 (ANF/CDD-95-126, Urodilatin). Es wird deutlich, daß das phosphorylierte CDD-32 bei entsprechender Konzentration deutlich geringeres Relaxationsvermögen besitzt als die nicht phosphorylierte Form. Zum Vergleich wird in Figur 7 auch das entsprechende Verhältnis für das Alpha-hANaP-Paar angegeben. Daraus wird ersichtlich, daß jeweils die phosphorylierten Verbindungen einander entsprechend geringere Aktivitäten als die nicht phosphorylierten Stammverbindungen aufweisen.

Die Erfindung wird anhand der folgenden Beispiele näher erläutert:

### Beispiel 1

Vorreinigung von Urin- und Serumproben (je 2 ml) über SEP-PAK Kapseln (C18-Cartrige, Waters Assoc., Millipore). Nach Aktivierung der kapseln (2 ml TriäthylaminMethanol, 20:80) erfolgt die Auftragung von 2 ml Probenvolumen und Elution mit 2 ml Aktivierungslösung.

Reaktionsansatz zur Phosphorylierung: 100 µl Reaktionsansatz enthalten 50 mM MOPS-Puffer (N-Morpholino-3-Propansulfonsäure), pH 6,8, 10 mM Magnesiumacetat, ca. 12,7 µg katalytische Untereinheit c-AMP-abhängige Proteinkinase (EC 2.7.1.37) aus Rinderherz (10 µl), Serumbzw. Urinprobe. Die Phosphorylierungsreaktion wird durch Zusatz von 2 mM ATP und [Gamma-³²P]ATP (10 µCi/20 nMol ATP) in 10 µl durch Mischen gestartet. Die Inkubation erfolgt bei 30°C 5 Minuten lang. Die Konzentration an katalytischer Untereinheit kann auch so gewählt werden, daß innerhalb von 5 bis 40 Minuten die vollständige Phosphorylierung stattfindet.

Die Phosphorylierungsreaktion wird durch Schockgefrieren in flüssigem Stickstoff beendet.

Die Auftrennung des phosphorylierten Reaktionsansatzes erfolgt durch HPLC über eine TSK-ODS-120T Säule der Firma Toyo Soda (300 x 7,8 mm ID). Zur Elution wird ein linearer Acetonitrilgradient (0 bis 80%) in 0,01 N HCl verwendet. Die Absorption wird bei 210 nm gemessen. Das gesammelte Fraktionsvolumen beträgt 1,5 ml. Der Einbau von ³²P wird in 0,5 ml Aliquots zusammen mit 2 ml Wasser im Szintillationszähler gemessen (1 Min.).

Als Referenzpeptide wurden synthetisch hergestelltes Cardiodilatin-28 (CDD-28, Bissendorf Peptides Co.) und -32 (Bachem) sowie CDD-88 aus Rinderherzvorhöfen für die Phosphorylierung verwendet (Hock, D. et al, J. Chromatography 397 (1987), 347-353).

### Beispiel 2

Eine weitere Reinigung der phosphorylierten Peptide durch Umkehrphasen-HPLC kann wie folgt durchgeführt werden: Die nach der Phosphorylierung vorliegende Reaktionsmischung wird auf eine RP-Säule aufgetragen (Micropore, C₁₈, 4,6 x 30 mm; Firma Applied Biosystems). Als Apparatur kann eine für die analytische HPLC geeignete Apparatur, wie sie von Applied Biosystems geliefert wird, verwendet werden. Die Apparatur ist ausgestattet mit zwei Pumpen, einem 1400 A-Lösungsmittelzuführungssystem, einem Spectroflow 491 Dynamic Mixer/Injector (0,2 ml Probenschleife) und einem 1783 Absorbents Detector Collector. Die Säule wird zunächst mit Puffer A, der 0,1%-ige TFA (Trifluoressigsäure) enthält, äquilibriert und danach mit steigender Konzentration an Acetonitril (Puffer B 70% Acetonitril/0,09% TFA) entwickelt. Die Fließgeschwindigkeit beträgt 1 ml/min und die Absorption wird bei 220 nm gemessen. Die Chromatographie findet bei Raumtemperatur statt. Phosphorylierte und unphosphorylierte Peptide eluieren unter isokratischen Bedingungen als einheitlicher Peak bei 24% Acetonitril-Konzentration. Die die phosphorylierten Peptide enthaltende Fraktion wird gesammelt, gefriergetrocknet und bei -70°C gelagert.

### Beispiel 3

### Reinigung der phosphorylierten Peptide mittels Kationenaustauscher-HPLC

Die Trennung der phosphorylierten Peptide, beispielsweise Alpha-hANaP, geschieht mittels einer Kationenaustauschersäule (4 x 50 mm) mit einer HPLC-Apparatur der Firma LKB, welche mit zwei Pumpen, einem 2152 Controller, einem 2151 Variable Wavelength Monitor und einem Reodyne Injector (1 ml Probenschleife) ausgestattet ist. Die Säule wird mit einem linearen Gradienten mit Natriumchlorid in 10 mM Dinatriumhydrogenphosphat (pH 5) mit einer Fließgeschwindigkeit von 0,7 ml/min entwickelt. Die Absorption wird bei 230 nm gemessen. Die Chromatographie wird bei Raumtemperatur durchgeführt. Die Anfangskonzentration an Natriumchlorid beträgt 100 mM. Die das phosphorylierte Alpha-hANaP enthaltenden Fraktionen werden durch Messung der enthaltenden Radioaktivität bestimmt. Die entsprechenden Fraktionen werden gesammelt, gefriergetrocknet und bei -70°C gelagert. Die Probe kann mittels einer weiteren Umkehrphasen-HPLC vom Salz befreit werden.

### Beispiel 4

### Bioaktivitätstests

Aortae von Neuseeland-Kaninchen wurden präpariert. Die Muskelspannung wurde an Muskelstreifen in einem Organbad, enthaltend physiologische Kochsalzlösung, gemessen. Die Streifen wurden vorkontrahiert mit einer Lösung von 10⁻⁶ M Norepinephrin, danach bei vollständiger Relaxation. Für den Assay wurde eine weitere Kontraktion mit 10⁻⁷ M Norepinephrin ausgelöst. Nachdem konstante Kontraktionsbedingungen erreicht waren, wurden die Peptide appliziert.

### Beispiel 5

### Effekte des phosphorylierten Alpha-hANaP auf Muskelrelaxation

Alpha-hANaP wurde gemäß Beispiel 1 in Anwesenheit von ATP und der katalytischen Untereinheit gemäß Beispiel 1 phosphoryliert. Zur Kontrolle wurde nicht phosphoryliertes Alpha-hANaP unter gleichen Bedingungen inkubiert, jedoch ohne Anwesenheit von ATP. Phosphoryliertes und nicht phosphoryliertes Alpha-hANaP wurde zu den vorkontrahierten Kaninchenaorta-Muskelstreifen gegeben, so daß sie in einer Endkonzentration von 0,3 x 10⁻⁹ M vorlagen. Unter dem Einfluß der nicht phosphorylierten Peptide relaxiert der Aorta-Muskelstreifen teilweise, während phosphoryliertes Alpha-hANaP nur einen vernachlässigbaren Effekt bewirkt. Schrittweise Zugabe von nicht phosphoryliertem, phosphoryliertem und wiederum nicht phosphoryliertem Alpha-hANaP läßt deutlich erkennen, daß lediglich nicht phosphoryliertes Alpha-hANaP eine signifikante relaxierende Wirkung im Vergleich zum phosphorylierten Peptid ausübt. Kehrt man die Zugabe der Peptide um, zunächst phosporyliertes, dann nicht phosphoryliertes und wieder phosphoryliertes Alpha-hANaP, so ergibt sich der entsprechend umgekehrte Relaxationseffekt.

### Beispiel 6

CDD-32 (Urodilatin) wird gemäß Beispiel 1 phosphoryliert und auf biologische Aktivität untersucht analog Beispiel 5. Urodilatin in nicht phosphoryliertem Zustand zeigt eine dosisabhängige Relaxationsaktivität mit einer stärkeren relaxierenden Wirkung bei höheren Konzentrationen. Verabreichung von phosphoryliertem Urodilatin in entsprechenden Konzentrationen bewirkt eine deutlich geringere Relaxation.

## Patentansprüche

1. Verbindung der Formel 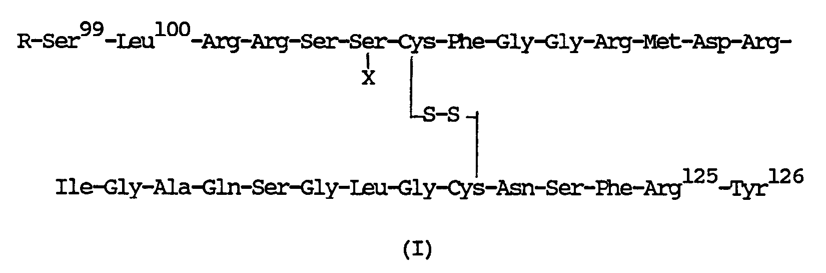 wobei X eine Phosphatgruppe oder eine Thiophosphatgruppe und R entweder ein -H Rest oder ein Peptidfragment aus der Aminosäuresequenz des Gamma-hANaP ist.

2. Verbindung nach Anspruch 1, dadurch gekennzeichnet, daß das Peptidfragment R die Aminosäuren 1-98, 39-98 oder 95-98 des Gamma-hANaP umfaßt.

3. Verbindung nach Anspruch 1 oder 2, dadurch gekennzeichnet, daß die Phosphatgruppe das Isotop ³²P enthält.

4. Verbindung nach Anspruch 1 oder 2, dadurch gekennzeichnet, daß die Thiophosphatgruppe die Isotope ³²P, ³⁵S oder beide radioaktive Isotope enthält.

5. Verfahren zur Herstellung der Verbindung gemäß Anspruch 1, wobei man die Verbindung mit der Formel 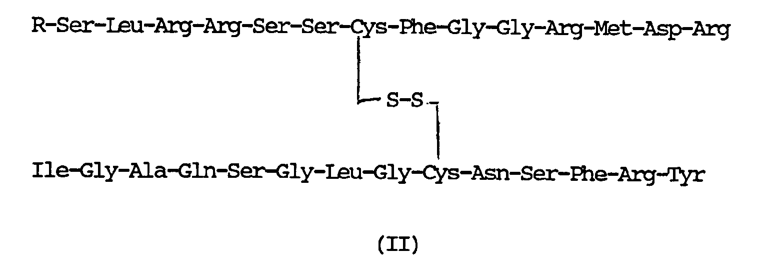 und R die in den Ansprüchen 1 und 2 genannten Bedeutungen hat, enzymatisch in Gegenwart von Nucleotiden oder (Gamma-thio)Nucleotiden umsetzt.

6. Verfahren nach Anspruch 5, dadurch gekennzeichnet, daß das Enzym c-AMP abhängige Proteinkinase oder deren katalytische Untereinheit ist und das Nucleotid ATP oder (Gamma-Thio)ATP ist.

7. Verfahren nach einem der Ansprüche 5 oder 6, dadurch gekennzeichnet, daß ATP mit ³²P oder ( Gamma-Thio)ATP mit ³²P und/oder ³⁵S markiert sind.

8. Verfahren zur Herstellung der Verbindung nach Anspruch 1 oder 2 durch chemische Totalsynthese gemäß der Merrifield Methode.

9. Arzneimittel enthaltend eine pharmazeutisch wirksame Dosis der Verbindung nach Anspruch 1 oder 2 sowie gegebenenfalls einen pharmazeutisch unbedenklichen Träger.

10. Arzneimittel nach Anspruch 9, wobei X die (Gamma-Thio)-ATP-Gruppe ist und für eine langsame Freisetzung des mindestens die Aminosäuresequenz des CDD-28 enthaltenden, biologisch aktiven Fragmentes sorgt.

11. Arzneimittel nach Anspruch 9 oder 10 zur Diagnose und Therapie von Hypertonie, Gefäß- und Herzkrankheiten, Hauterkrankungen mit Störungen der Schweißsekretion, des cardiovaskulären Schocks, von Erkrankungen der Nieren und -Nebennierenrinde, Erkrankungen des Gastrointestinaltraktes, insbesondere Motilitätsstörungen, des Hirnödems, des Glaukoms, spastischer Coronaropathien sowie von neurologischen Erkrankungen.

12. Arzneimittel nach Anspruch 9 oder 10 zur Behandlung von akuter Niereninsuffizienz, nephrotischem und nephritischem Syndrom, terminaler Niereninsuffizienz, akuter Herzinsuffizienz, Ascites, generalisierten Ödemen, Lungenödem, Hirnödem, primären und sekundären Lymphödemen, Hydrothorax, Glaukom, Vena-cava-Stenose, Hypoproteinämie, Hörsturz, essentieller und renaler Hypertonie, maligner Hypertonie, EPH-Gestose wie Schwangerschaftshypertonie, Schwangerschafts-Nephrose, zerebralem Natriumspeichersyndrom, Gefäßspasmen wie Morbus Raynaud, Angina abdominalis, Koronarspasmen, koronarer Herzkrankheit, vasomotorischen Kopfschmerzen, Kopfschmerzen bei Hypertonie, Bing-Horton-Syndrom Migräne, Durchblutungsstörungen im Vertebralis-Basilaris-Strombahngebiet, Störungen des Renin-Angiotensin-Systems, primärem und sekundärem Hyperaldosteronismus, reninsezernierenden Tumoren, Phäochromozytom, Bartters Syndrom, Schwartz-Bartter-Syndrom, Pankreasinsuffizienz, primärer Schweißdrüseninsuffizienz wie Anhidrosis, Miktions- und Defäkationsstörungen, Hypophysenvorderlappendysregulation, cardio-vaskulären Nebenwirkungen bei der Therapie psychiatrischer Erkrankungen sowie Agitation mit Catecholaminerhöhung.

13. Arzneimittel nach Anspruch 9 oder 10 zur unterstützenden Behandlung bei Herztransplantationen, "PEEP-Beatmung" (einer mechanischen Beatmung mit positivem endexpiratorischem Druck) sowie Bypass-Operationen.

14. Arzneimittel nach Anspruch 9 oder 10 zur Behandlung von Komplikationen nach Einpflanzung eines künstlichen Herzens.

15. Arzneimittel nach Anspruch-9 oder 10 als Diuretikum zur Behandlung von multiplen Erkrankungen wie Gicht, kombiniert mit Hypertonus/Herzinsuffizienz sowie Diabetes mit Hypertonus/Herzinsuffizienz.

16. Arzneimittel nach Anspruch 9 oder 10 als Diuretikum in Kombination mit Cephalosporinen, Aminoglykosiden oder Antikoagulantien.

17. Arzneimittel nach Anspruch 9 oder 10 zur Prophylaxe von akutem Nierenversagen nach Nierentransplantationen und Behandlung mit nephrotoxischen Substanzen wie Cyclosporin, Cisplatin und/oder Ifosfamid.

18. Arzneimittel nach Anspruch 9 oder 10 zur Prophylaxe des akuten Nierenversagens, postoperativ oder nach hypertonen Krisen sowie nach Gabe von Kontrastmitteln bei Patienten mit eingeschränkter Nierenfunktion.

19. Arzneimittel nach Anspruch 9 oder 10 als Diagnostikum zur Differentialdiagnose von Endothel-Veränderungen.

20. Arzneimittel nach Anspruch 9 oder 10, dadurch gekennzeichnet, daß es 10 ng bis 50 µg der Verbindung gemäß Anspruch 1 oder 2 pro Dosiseinheit enthält.

21. Arzneimittel nach Anspruch 9 oder 10 mit vasodilatorischer Wirkung, dadurch gekennzeichnet, daß es die Verbindung gemäß Anspruch 1 oder 2 als Wirkstoff enthält.

22. Verwendung der Verbindung nach einem der Ansprüche 1 oder 2 zur Herstellung von Arzneimitteln wie Injektions- oder Infusionslösungen zur Behandlung von akuter Niereninsuffizienz, nephrotischem und nephritischem Syndrom, terminaler Niereninsuffizienz, akuter Herzinsuffizienz, Ascites, generalisierten Ödemen, Lungenödem, Hirnödem, primären und sekundären Lymphödemen, Hydrothorax, Glaukom, Vena-cava-Stenose, Hypoproteinämie, Hörsturz, essentieller und renaler Hypertonie, maligner Hypertonie, EPH-Gestose wie Schwangerschaftshypertonie, Schwangerschafts-Nephrose, zerebralem Natriumspeichersyndrom, Gefäßspasmen wie Morbus Raynaud, Angina abdominalis, Koronarspasmen, koronarer Herzkrankheit, vasomotorischen Kopfschmerzen, Kopfschmerzen bei Hypertonie, Bing-Horton-Syndrom Migräne, Durchblutungsstörungen im Vertebralis-Basilaris-Strombahngebiet, Störungen des Renin-Angiotensin-Systems, primärem und sekundärem Hyperaldosteronismus, reninsezernierenden Tumoren, Phäochromozytom, Bartters Syndrom, Schwartz-Bartter-Syndrom, Pankreasinsuffizienz, primärer Schweißdrüseninsuffizienz wie Anhidrosis, Miktions- und Defäkationsstörungen, Hypophysenvorderlappendysregulation, cardio-vaskulären Nebenwirkungen bei der Therapie psychiatrischer Erkrankungen sowie Agitation mit Catecholaminerhöhung.

23. Verwendung der Verbindung nach einem der Ansprüche 1 oder 2 zur Herstellung von Arzneimitteln wie Injektions- oder Infusionslösungen zur unterstützenden Behandlung bei Herztransplantationen, "PEEP-Beatmung" (einer mechanischen Beatmung mit positivem endexpiratorischem Druck) sowie Bypass-Operationen.

24. Verwendung der Verbindung nach einem der Ansprüche 1 oder 2 zur Herstellung von Arzneimitteln wie Injektions - oder Infusionslösungen zur Behandlung von Komplikationen nach Einpflanzung eines künstlichen Herzens.

25. Verwendung der Verbindung nach einem der Ansprüche 1 oder 2 zur Herstellung eines Diuretikums zur Behandlung von multiplen Erkrankungen wie Gicht, kombiniert mit Hypertonus/Herzinsuffizienz sowie Diabetes mit Hypertonus/ Herzinsuffizienz.

26. Verwendung der Verbindung nach einem der Ansprüche 1 oder 2 zur Herstellung eines Diuretikums in Kombination mit Cephalosporinen, Aminoglykosiden oder Antikoagulantien.

27. Verwendung der Verbindung nach einem der Ansprüche 1 oder 2 zur Herstellung von Arzneimitteln wie Injektions- oder Infusionslösungen zur Prophylaxe von akutem Nierenversagen nach Nierentransplantationen und Behandlung mit nephrotoxischen Substanzen wie Cyclosporin, Cisplatin und/oder Ifosfamid.

28. Verwendung der Verbindung nach einem der Ansprüche 1 oder 2 zur Herstellung von Arzneimitteln wie Injektions- oder Infusionslösungen zur Prophylaxe des akuten Nierenversagens, postoperativ oder nach hypertonen Krisen sowie nach Gabe von Kontrastmitteln bei Patienten mit eingeschränkter Nierenfunktion.

29. Verwendung der Verbindung gemäß einem der Ansprüche 1 bis 4 in einem Verfahren zur qualitativen und/oder quantitativen Messung des Gehaltes an pre-pro-Gamma-hANaP und/ oder zumindest das Fragment CDD-28, CDD-32 und/oder CDD-88 enthaltenden Fragmenten des pre-pro-Gamma-hANaP in Körperflüssigkeiten wie Serum, Urin und Gewebehomogenaten.

30. Verwendung der Verbindung nach einem der Ansprüche 1 bis 4 zur Herstellung eines Diagnostikums zur Differentialdiagnose von Endothel-Veränderungen.

31. Verfahren zur spezifischen Bestimmung der Verbindung gemäß Anspruch 1 oder 2, dadurch gekennzeichnet, daß man nach dem Prinzip des Immunoassays arbeitet und einen Antikörper, der gegen die entsprechende Verbindung gerichtet ist, verwendet.

## Claims

1. A compound having the formula 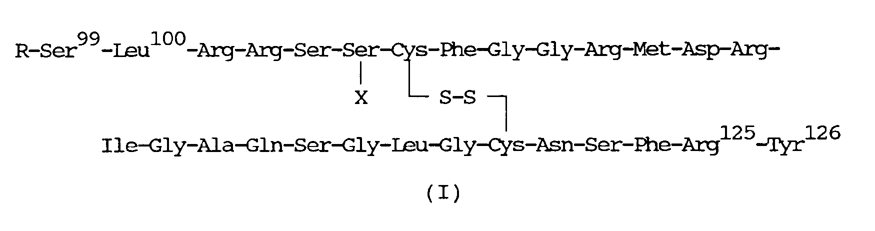 wherein X is a phosphate group or thiophosphate group and R is either a residue -H or a peptide fragment from the amino acid sequence of gamma-hANaP.

2. The compound according to claim 1, characterized in that the peptide fragment R includes the amino acids 1-98, 39-98 or 95-98 of the gamma-hANaP.

3. The compound according to claim 1 or claim 2, characterized in that the phosphate group contains the isotope ³²P.

4. The compound according to claim 1 or claim 2, characterized in that the thiophosphate group contains the isotopes ³²P, ³⁵S or both radioactive isotopes.

5. A process for preparing the compound according to claim 1, wherein the compound of the formula 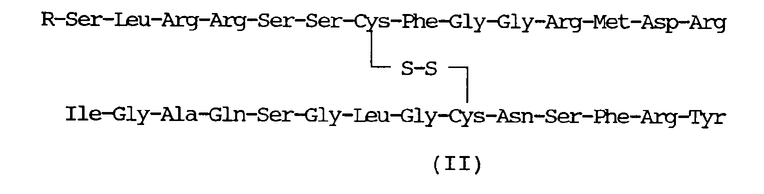 wherein
R has the meanings as set forth in claims 1 and 2, is enzymatically reacted in the presence of nucleotides or (gamma-thio)nucleotides.

6. The process according to claim 5, characterized in that the enzyme is c-AMP-dependent proteinkinase or its catalytic subunits, and the nucleotide is ATP or (gamma-thio)ATP.

7. The process according to anyone of claims 5 or 6, characterized in that ATP has been labelled with ³²P or (gamma-thio)ATP has been labelled with ³²P and/or ³⁵S.

8. A process for preparing the compound according to claims 1 or 2 by chemical total synthesis according to the Merrifield method.

9. A medicament, containing a pharmaceutically effective dose of the compound according to claims 1 or 2 as well as optionally a pharmaceutically acceptable carrier.

10. The medicament according to claim 9, wherein X is the (gamma-thio)ATP group and provides a slow release of the biologically active fragment containing at least the amino acid sequence of the CDD-28.

11. The medicament according to claims 9 or 10 for diagnostics and therapy of hypertonia, vessel and heart diseases, skin diseases including perspiration disorders, cardiovascular shock, renal and adrenal cortical disorders, diseases of the gastro-intestinal tract, and more particularly motility disorders, brain edemae, glaucoma, spastic coronaropathies and neurological diseases.

12. The medicament according to claims 9 or 10 for the treatment of acute renal insuffiency, nephrotic and nephritic syndrome, terminal renal insufficiency, acute coronary insufficiency, ascites, generalized edemae, lung edema, brain edema, primary and secondary lymph edemae, hydrothorax, glaucoma, vena cava stenosis, hypoproteinemia, hearing impairment, essential and renal hypertonia, malignant hypertonia, EPH gestosis such as hypertonia of pregnancy, nephrosis of pregnancy, cerebral sodium storage syndrome, vessel spasms such as Morbus Raynaud, angina abdominalis, coronary spasms, coronary heart disease, vasomotor headache, headache upon hypertonia, Bing-Horton syndrome migraine, blood circulation disorders in the vertebralis-basilaris flow path region, disorders of the renin-angiotensin system, primary and secondary hyperaldosteronism, renin-secernent tumors, phaeochromocytoma, Bartter's syndrome, Schwartz-Bartter's syndrome, pancreas insufficiency, primary sweat gland insufficiency such as anhidrosis, miction and defecation disorders, dysregulation of the anterior lobe of the pituitary gland, cardiovascular side-effects in the therapy of psych(iatr)ic diseases and agitation with catechol increase.

13. The medicament according to claims 9 or 10 for the supporting treatment upon heart transplantations, "PEEP-respiration" (an artificial respiration with positive end expiratory pressure) as well as by-pass operations.

14. The medicament according to claims 9 or 10 for the treatment of complications after implantation of an artificial heart.

15. The medicament according to claims 9 or 10 as a diuretic for the treatment of multiple diseases such as gout combined with hypertonia/heart insufficiency, as well as diabetes with hypertonia/heart insufficiency.

16. The medicament according to claims 9 or 10 as a diuretic in combination with cephalosporins, aminoglycosides or anticoagulants.

17. The medicament according to claims 9 or 10 for the prophylaxis of an acute renal failure after kidney transplantations and treatment with nephrotoxic substances such as cyclosporin, cisplatin and/or ifosfamid.

18. The medicament according to claims 9 or 10 for the prophylaxis of an acute kidney failure which may occur postoperative or after hypertonic crisis as well as after dosis of contrast medium for a patient suffering from limited function of the kidney.

19. The medicament according to claims 9 or 10 as a diagnostic for differential diagnostics of endothelial changes.

20. The medicament according to claims 9 or 10, characterized in that it contains from 10 ng to 50 µg of the compound according to claims 1 or 2 per dosage unit.

21. The medicament according to claims 9 or 10 having a vasodilative effect, characterized in that it contains the compound according to claims 1 or 2 as the active ingredient.

22. Use of the compound according to anyone of claims 1 or 2 for the preparation of medicaments such as injection or infusion solutions for the treatment of acute renal insuffiency, nephrotic and nephritic syndrome, terminal renal insufficiency, acute coronary insufficiency, ascites, generalized edemae, lung edema, brain edema, primary and secondary lymph edemae, hydrothorax, glaucoma, vena cava stenosis, hypoproteinemia, hearing impairment, essential and renal hypertonia, malignant hypertonia, EPH gestosis such as hypertonia of pregnancy, nephrosis of pregnancy, cerebral sodium storage syndrome, vessel spasms such as Morbus Raynaud, angina abdominalis, coronary spasms, coronary heart disease, vasomotor headache, headache upon hypertonia, Bing-Horton syndrome migraine, blood circulation disorders in the vertebralis-basilaris flow path region, disorders of the renin-angiotensin system, primary and secondary hyperaldosteronism, renin-secernent tumors, phaeochromocytoma, Bartter's syndrome, Schwartz-Bartter's syndrome, pancreas insufficiency, primary sweat gland insufficiency such as anhidrosis, miction and defecation disorders, dysregulation of the anterior lobe of the pituitary gland, cardiovascular side-effects in the therapy of psych(iatr)ic diseases and agitation with catecholamine increase.

23. Use of the compound according to anyone of claims 1 or 2 for the preparation of medicaments such as injection or infusion solutions for the supporting treatment upon heart transplantations, "PEEP-respiration" (an artificial respiration with positive end expiratory pressure) as well as by-pass surgery.

24. Use of the compound according to anyone of claims 1 or 2 for the preparation of medicaments such as injection or infusion solutions for the treatment of complications after implantation of an artificial heart.

25. Use of the compound according to anyone of claims 1 or 2 for the preparation of a diuretic for the treatment of multiple diseases such as gout combined with hypertonia/heart insufficiency, as well as diabetes with hypertonia/heart insufficiency.

26. Use of the compound according to anyone of claims 1 or 2 for the preparation of a diuretic in combination with cephalosporins, aminoglycosides or anticoagulants.

27. Use of the compound according to anyone of claims 1 or 2 for the preparation of medicaments such as injection or infusion solutions for the prophylaxis of an acute renal failure after kidney transplantations and treatment with nephrotoxic substances such as cyclosporin, cisplatin and/or ifosfamid.

28. Use of the compound according to anyone of claims 1 or 2 for the preparation of medicaments such as injection or infusion solutions for the prophylaxis of an acute kidney failure which may occur postoperative or after hypertonic crisis as well as after dosis of contrast medium for a patient suffering from limited function of the kidney.

29. Use of the compound according to anyone of claims 1 to 4 in a method for the qualitative and/or quantitative measurement of the content of the pre-pro-gamma-hANaP and/or fragments containing at least one of the fragments CDD-28, CDD-32 and/or CDD-88 of the pre-pro-gamma-hANaP in body fluids such as serum, urine and tissue homogenates.

30. Use of the compound according to anyone of claims 1 to 4 for the preparation of a diagnostic for differential diagnostics of endothelial changes.

31. A method for the specific determination of the compound according to claims 1 or 2, characterized in that the principle of immunoassay is employed and an antibody directed against said compound is used.

## Revendications

1. Composé répondant à la formule : 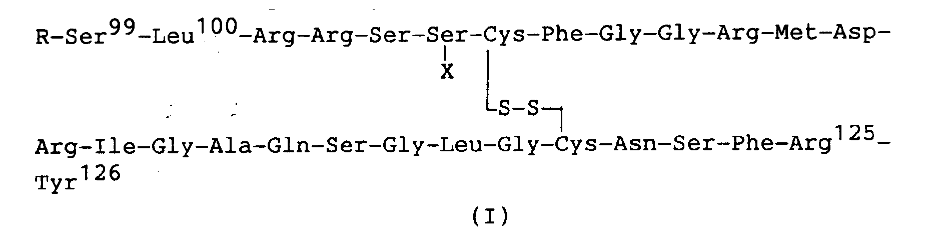 dans laquelle X est un groupe phosphate ou un groupe thiophosphate et R est un reste -H ou un fragment peptidique de la séquence d'amino-acides du gamma-hANaP.

2. Composé selon la revendication 1, caractérisé en ce que le fragment peptidique R contient les amino-acides 1-98, 39-98 ou 95-98 du gamma-hANaP.

3. Composé selon la revendication 1 ou 2, caractérisé en ce que le groupe phosphate contient l'isotope ³²p.

4. Composé selon la revendication 1 ou 2, caractérisé en ce que le groupe thiophosphate contient les isotopes ³²P, ³⁵S ou ces deux isotopes radioactifs.

5. Procédé de préparation du composé selon la revendication 1, dans lequel on fait réagir enzymatiquement le composé de formule 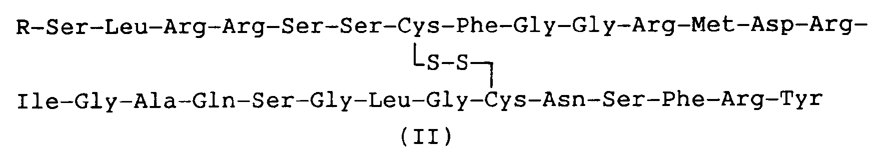 où R a les significations indiquées dans les revendications 1 et 2, en présence de nucléotides ou de (gamma-thio)nucléotides.

6. Procédé selon la revendication 5, caractérisé en ce que l'enzyme est une protéine-kinase AMPc-dépendante ou la sous-unité catalytique de celle-ci, et la nucléotide est de l'ATP ou du (gamma-thio)ATP.

7. Procédé selon la revendication 5 ou 6, caractérisé en ce que l'ATP est marqué au ³²P ou le (gamma-thio) ATP est marqué au ³²P et/ou au ³⁵S.

8. Procédé de préparation du composé selon la revendication 1 ou 2 au moyen d'une synthèse chimique totale d'après le procédé de Merrifield.

9. Médicament contenant une dose pharmaceutiquement active du composé selon la revendication 1 ou 2, ainsi qu'éventuellement un excipient pharmaceutiquement neutre.

10. Médicament selon la revendication 9, dans lequel X est le groupe (gamma-thio)-ATP et garantit une libération lente du fragment biologiquement actif contenant au moins la séquence d'amino-acides du CDD-28.

11. Médicament selon la revendication 9 ou 10 pour le diagnostic et le traitement de l'hypertonie, des maladies cardio-vasculaires, des maladies de la peau avec disfonctionnement de la sécrétion sudorale, du choc cardiovasculaire, des maladies des reins et des corticosurrénales, des maladies de l'appareil digestif, et plus spécialement les troubles de la motilité, de l'oedème du cerveau, du glaucome, des coronaropathies spasmotiques, ainsi que des maladies neurologiques.

12. Médicament selon la revendication 9 ou 10 pour le traitement de l'insuffisance rénale aiguë, du syndrome néphrotique ou néphrétique, de l'insuffisance rénale terminale, de l'insuffisance cardiaque aiguë, de l'ascite, des oedèmes généralisés, de l'oedème pulmonaire, de l'oedème du cerveau, des lymphoedèmes primaires et secondaires, de l'hydrothorax, du glaucome, de la sténose de la veine cave, de l'hypoprotéinémie, d'une diminution brutale de l'audition, de l'hypertonie essentielle et rénale, de l'hypertonie maligne, de la gestose EPH comme l'hypertonie de la grossesse, de la néphrose de la grossesse, du syndrome cérébral d'accumulation de sodium, des spasmes vasculaires comme le Morbus Raynaud, l'angine de poitrine, des spasmes coronariens, des maladies cardiocoronariennes, des céphalées avec troubles vasomoteurs, des céphalées avec hypertonie, des migraines avec syndrome de Bing-Horton, des troubles de la circulation sanguine dans la région d'irrigation vertébro-basilaire, des disfonctionnements du système rénine-angiotensine, de l'hyperaldostéronisme primaire ou secondaire, des tumeurs avec sécrétion de rénine, des phéochromocytomes, du syndrome de Bartter, du syndrome de Schwartz-Bartter, de l'insuffisance pancréatique, de l'insuffisance primaire des glandes sudoripares comme l'anhydrose, des troubles de la miction et de la défécation, d'un disfonctionnement du lobe antérieur de l'hypophyse, des effets secondaires cardio-vasculaires lors du traitement des maladies psychiatriques, ainsi que d'une agitation avec augmentation du taux de catécholamine.

13. Médicament selon la revendication 9 ou 10, pour le traitement d'appoint lors de transplantations cardiaques, d'une ventilation artificielle "PEEP" (ventilation artificielle avec pression positive résiduelle expiratoire), ainsi que d'opérations de pontage.

14. Médicament selon la revendication 9 ou 10 pour le traitement de complications survenant après l'implantation d'un coeur arti-artificiel.

15. Médicament selon la revendication 9 ou 10, comme diurétique pour le traitement de maladies multiples, comme la goutte associée à une insuffisance hypertonique/ cardiaque, ainsi que le diabète associé à une insuffisance hypertonique/cardiaque.

16. Médicament selon la revendication 9 ou 10, comme diurétique en association avec des céphalosporines, des aminoglucosides ou des anticoagulants.

17. Médicament selon la revendication 9 ou 10, pour la prophylaxie d'une défaillance rénale aiguë après des transplantations rénales et un traitement avec des substances néphrotoxiques comme la cyclosporine, le cisplatine et/ou l'ifosfamide.

18. Médicament selon la revendication 9 ou 10, pour la prophylaxie de la défaillance rénale aiguë postopératoire ou après des crises hypertoniques, ainsi qu'après l'administration de produits de contraste chez des patients à fonction rénale limitée.

19. Médicament selon la revendication 9 ou 10, comme agent diagnostique pour le diagnostic différentiel des modifications de l'endothélium.

20. Médicament selon la revendication 9 ou 10, caractérisé en ce qu'il contient 10 ng à 50 µg du composé selon la revendication 1 ou 2 par dose unitaire.

21. Médicament selon la revendication 9 ou 10, ayant un effet vasodilatateur, caractérisé en ce qu'il contient le composé selon la revendication 1 ou 2 comme principe actif.

22. Utilisation du composé selon la revendication 1 ou 2 pour la préparation de médicaments comme des solutions pour injections ou perfusions pour le traitement de l'insuffisance rénale aiguë, du syndrome néphrotique ou néphrétique, de l'insuffisance rénale terminale, de l'insuffisance cardiaque aiguë, de l'ascite, des oedèmes généralisés, de l'oedème pulmonaire, de l'oedème du cerveau, des lymphoedèmes primaires et secondaires, de l'hydrothorax, du glaucome, de la sténose de la veine cave, de l'hypoprotéinémie, d'une diminution brutale de l'audition, de l'hypertonie essentielle et rénale, de l'hypertonie maligne, de la gestose EPH comme l'hypertonie de la grossesse, de la néphrose de la grossesse, du syndrome cérébral d'accumulation de sodium, des spasmes vasculaires comme le Morbus Raynaud, de l'angine de poitrine, des spasmes coronariens, des maladies cardiocoronariennes, des céphalées avec troubles vasomoteurs, des céphalées jar hypertonie, des migraines avec syndrome de Bing-Horton, des troubles de la circulation sanguine dans la région d'irrigation vertébro-basilaire, des disfonctionnements du système rénine-angiotensine, de l'hyperaldostéronisme primaire ou secondaire, des tumeurs avec sécrétion de rénine, des phéochromocytomes, du syndrome de Bartter, du syndrome de Schwartz-Bartter, de l'insuffisance pancréatique, de l'insuffisance primaire des glandes sudoripares comme l'anhydrose, des troubles de la miction et de la défécation, d'un disfonctionnement du lobe antérieur de l'hypophyse, des effets secondaires cardiovasculaires lors du traitement des maladies psychiatriques, ainsi que d'une agitation avec augmentation du taux de catécholamine.

23. Utilisation du composé selon la revendication 1 ou 2 pour la préparation de médicaments comme des solutions pour injections ou perfusions pour le traitement d'appoint lors de transplantations cardiaques et d'une ventilation artificielle "PEEP" (ventilation artificielle avec pression positive résiduelle expiratoire), ainsi que d'opérations de pontage.

24. Utilisation du composé selon la revendication 1 ou 2 pour la préparation de médicaments comme des solutions pour injections ou perfusions pour le traitement des complications après l'implantation d'un coeur artificiel.

25. Utilisation du composé selon la revendication 1 ou 2 pour la préparation d'un diurétique pour le traitement de maladies multiples comme la goutte associée à une insuffisance hypertonique/cardiaque, ainsi que le diabète associé à une insuffisance hypertonique/cardiaque.

26. Utilisation du composé selon la revendication 1 ou 2 pour la préparation d'un diurétique en association avec des céphalosporines, des aminoglucosides ou des anticoagulants.

27. Utilisation du composé selon la revendication 1 ou 2 pour la préparation de médicaments comme des solutions pour injections ou perfusions pour la prophylaxie d'une défaillance rénale aiguë après des transplantations rénales et un traitement avec des substances néphrotoxiques comme la cyclosporine, le cisplatine et/ou l'ifosfamide.

28. Utilisation du composé selon la revendication 1 ou 2 pour la préparation de médicaments comme des solutions pour injections ou perfusions pour la prophylaxie de la défaillance rénale aiguë postopératoire ou après des crises hypertoniques, ainsi qu'après l'administration de produits de contraste chez des patients à fonction rénale limitée.

29. Utilisation du composé selon une des revendications 1 à 4 dans un procédé de mesure qualitative et/ou quantitative de la teneur en pré-pro-gamma-hANaP et/ou en fragments du pré-pro-gamma-hANaP contenant au moins le fragment CDD-28, CDD-32 et/ou CDD-88, dans des liquides corporels comme le sérum, l'urine et des homogenéisats des tissus.

30. Utilisation du composé selon une des revendications 1 à 4 pour la préparation d'un agent diagnostique pour le diagnostic différentiel des modifications de l'endothélium.

31. Procédé de détermination spécifique du composé selon la revendication 1 ou 2, caractérisé en ce qu'on travaille d'après le principe de l'immuno-essai et on utilise un anticorps qui est dirigé contre le composé correspondant.
